# EUROPEAN PATENT SPECIFICATION

(11) **EP 3 699 676 B1**
(45) Date of publication and mention of the grant of the patent: **10.04.2024**
(21) Application number: 18868421.1
(22) Date of filing: 17.10.2018
(51) Int. Cl.: G02C 7/04, B29D 11/02, B29D 11/00, B29C 39/26, B29C 39/24, B29C 39/12, A61F 2/16

(54) **METHOD FOR MANUFACTURING OPHTHALMIC LENS IN WHICH MEMBER IS EMBEDDED**
VERFAHREN ZUR HERSTELLUNG EINER OPHTALMISCHEN LINSE MIT EINGEBETTETER KOMPONENTE
PROCÉDÉ DE FABRICATION DE LENTILLE OPHTALMIQUE DANS LAQUELLE EST INCORPORÉ UN ÉLÉMENT

(30) Priority: 18.10.2017 JP 2017202114
(43) Date of publication of application: 26.08.2020
(73) Proprietor: Seed Co., Ltd., Tokyo 113-8402 (JP)
(72) Inventor: JONIN, Kunio, Tokyo 113-8402 (JP); ARAI, Ritsuko, Tokyo 113-8402 (JP); FUKUDA, Takeshi, Tokyo 113-8402 (JP)
(74) Representative: Schulz Junghans Patentanwälte PartGmbB
(86) International application number: PCT/JP2018/038590
(87) International publication number: WO 2019/078231

(56) References cited:
- EP-A2- 0 488 627
- WO-A1-2014/073568
- JP-A- 2007 256 923
- JP-A- 2014 134 795
- US-B1- 6 217 171

## Description

### Cross-Reference to Related Application

The present application claims the benefit of priority to Japanese Patent Application No. 2017-202114, filed Oct 18, 2017.

### Technical Field

The present invention relates to a method of producing an ophthalmic lens having a component embedded therein.

### Background of the Invention

Ophthalmic lenses were developed originally as devices for correcting vision. In particular, contact lenses with added value have recently been provided, including so-called fashion contact lenses having an annular tinted area for altering the iris color and textures and measuring contact lenses equipped with a self-supplied energy device that enables monitoring and/or examination of biological functions.

Such fashion contact lenses and measuring contact lenses can be formed by embedding within a contact lens substrate a component such as iris-patterned film and an energy device. For example, a method for forming multi-piece insert device having an electronic component secured in proximity to the surface of an ophthalmic device (see, Patent Document 1 listed below) and a method for manufacturing a device with an insert having a colorant pattern (see, Patent Document 2 listed below) are known.

### Prior-Art Documents

### Patent Documents

Patent Document 1: JP-A-2014-134795
Patent Document 2: Pamphlet of WO2014/073568
Patent Document 3: US 6 217 171 B1 discloses the preamble of independent claims 1 and 2.

### Summary of the Invention

### Technical Problem

For contact lenses with an embedded component, the location of the embedded component within a contact lens affects the safety of the contact lens for wearing. If the embedded component is partially or fully exposed on the surface of a contact lens, it may cause damage to eye tissue. Also, if the embedded component interferes with an optical zone, there arises a concern that the optical characteristics of the contact lens may be adversely affected.

The method described in Patent Document 1 uses a mold that has a sticking feature for sticking together a male mold and female mold. The mold, however, does not include any positioning feature for positioning a component to be embedded (i.e., embedded component). As a result, the method described in Patent Document 1 may result in shifting of the embedded components due to the pressure exerted upon mating of the male mold. The method described in Patent Document 2 controls the thickness of first and second components in order to prevent drying of the contact lens and to thereby minimize defects resulting from deformation caused by drying. While the method can control the thickness of the contact lens substrate that carries an embedded colored film, the method uses no particular features for positioning the embedded colored film.

As described above, no traditional methods for forming contact lenses with embedded components take into consideration the shifting of the embedded components that occurs during formation of the contact lenses and thus, the concern about the effect on the resulting optical characteristics still remains.

Accordingly, it is an objective of the present invention to provide a method of producing an ophthalmic lens with embedded components that, while based on common ophthalmic lens-forming techniques that use molds, does not cause floating or shifting of the embedded components during mating of molds, thus achieving improved position stability of the embedded components.

### Solution to Problem

The present inventors have conducted extensive studies to find a way to improve the position stability of embedded components and, through trials and failures in an effort to devise molds used for forming ophthalmic lenses and methods for forming the ophthalmic lenses using the molds, have come to focus on the use of specific molds to form impressions. The present inventors have envisioned that pre-forming a recess conforming to the shape of a component to be embedded on the surface of an ophthalmic lens may achieve stable positioning of the component to be embedded.

Over the course of our continuous effort to further develop the above-described concept, the present inventors have ultimately devised a method for forming an ophthalmic lens by using a first male or female mold having on an ophthalmic lens-side surface of the mold a protrusion conforming to the shape of a component to be embedded, a second female or male mold that corresponds to the first male or female mold and has no protrusion thereon, and a third male or female mold that corresponds to the second female or male mold and has no protrusion thereon. In particular, the present inventors have successfully formed an ophthalmic lens that has a component embedded in a desired position by subjecting an ophthalmic lens material to a first copolymerization reaction using the first male or female mold having the protrusion thereon as well as the second male or female mold having no protrusion to obtain on a surface of the second female or male mold a first layer of the ophthalmic lens having a recess formed thereon corresponding to the protrusion, placing the component in the formed recess, and subsequently, subjecting an additional ophthalmic lens material to a second copolymerization reaction using a third male or female mold with no protrusion to form the ophthalmic lens with the component embedded in the desired position. It is these findings and successful examples that have led to the completion of the present invention.

Accordingly, the present invention provides a method according to claim 1 for manufacturing a contact lens having a component embedded therein, the method comprising the steps of:
(A1) mating a first male mold with a first female mold with a first ophthalmic lens material injected therein, and subjecting the first ophthalmic lens material to a first copolymerization reaction to form a first layer of the ophthalmic lens on a concave forming surface of the first female mold, wherein the first male mold has a convex forming surface having a protrusion thereon conforming to the shape of the component to be embedded within the ophthalmic lens, and the first female mold has the concave forming surface corresponding to the first male mold;
(B1) separating the first male mold from the first female mold, placing the component to be embedded in a recess in the first layer of the ophthalmic lens that corresponds to the protrusion, and injecting a second ophthalmic lens material onto the concave forming surface of the first female mold;
(C1) mating a second male mold with the first female mold with the second ophthalmic lens material injected therein, the second male mold having a convex forming surface corresponding to the first female mold, and subjecting the second ophthalmic lens material to a second copolymerization reaction to form a second layer of the ophthalmic lens over the concave forming surface of the first female mold; and
(D1) separating the second male mold from the first female mold to obtain the ophthalmic lens having the component embedded between the second layer and the first layer.

Accordingly, the present invention provides a method according to claim 2 for manufacturing a contact lens having a component embedded therein, the method comprising the steps of:
(A2) mating a third male mold with a second female mold with a third ophthalmic lens material injected therein, and subjecting the third ophthalmic lens material to a first copolymerization reaction to form a first layer of the ophthalmic lens on a convex forming surface of the third male mold, wherein the second female mold has a concave forming surface having a protrusion thereon conforming to the shape of the component to be embedded within the ophthalmic lens, and the third male mold has the convex forming surface corresponding to the second female mold;
(B2) separating the second female mold from the third male mold, placing the component to be embedded in a recess in the first layer of the ophthalmic lens corresponding to the protrusion, and injecting a fourth ophthalmic lens material onto a concave forming surface of a third female mold that corresponds to the third male mold;
(C2) mating the third male mold with the third female mold with the fourth ophthalmic lens material injected therein, and subjecting the fourth ophthalmic lens material to a second copolymerization reaction to form a second layer of the ophthalmic lens on the first layer of the ophthalmic lens on the convex forming surface of the third male mold; and
(D2) separating the third male mold from the third female mold to obtain the ophthalmic lens with the component embedded between the second layer and the first layer.

In the method for manufacturing an ophthalmic lens having a component embedded therein, the ratio of the thickness of the first layer at its center to the thickness of the second layer at its center is preferably in the range of 1:0.1 to 1:25.

In the method for manufacturing an ophthalmic lens having a component embedded therein, the first and the second layers are formed of the same material.

In the method for manufacturing an ophthalmic lens having a component embedded therein, the first and the second layers are formed of different materials.

### Advantageous Effects of Invention

According to the present invention, the ophthalmic lenses having a component embedded in a desired position can be formed in a reliable fashion. The ophthalmic lenses obtained according to the present invention do not cause damage to the eye tissue when wearing them since the embedded components are not exposed on the surface of the ophthalmic lens.

In addition, the ophthalmic lenses obtained according to the present invention do not have adverse effects on the optical characteristics of the ophthalmic lens since the embedded components do not interfere with the optical zone. By pre-forming an impression of a component to be embedded, the ophthalmic lens of the present invention allows precise embedding of any component, thus allowing embedding of a more precise, sophisticated component with more complicated geometry. Accordingly, the ophthalmic lens is expected to find wide applications in cosmetic and medical uses.

### Brief Description of Drawings

FIG. 1 is a diagram illustrating a first embodiment of a mold for use in the present invention.
FIG. 2 is a schematic diagram of a production method of an ophthalmic lens according to the first embodiment of the present invention.
FIG. 3 is a diagram illustrating a second embodiment of the mold for use in the present invention.
FIG. 4 is a schematic diagram of a production method of an ophthalmic lens according to the second embodiment of the present invention.
FIG. 5A is a schematic diagram illustrating an ophthalmic lens with a chip-shaped (rectangular) measurement device as the embedded component of the present invention inserted therein.
FIG. 5B is a schematic diagram illustrating an ophthalmic lens with a chip-shaped (circular) measurement device as the embedded component of the present invention inserted therein.
FIG. 6 is a schematic diagram illustrating an ophthalmic lens with an annular measurement device as the embedded component of the present invention inserted therein.

### Detailed Description of the Invention

While a method for forming an ophthalmic lens with an embedded component in accordance with the present invention (which may be referred to as "method of the present invention," hereinafter) will now be described in further details, the scope of the present invention is not limited to what is described in this section.

The method of the present invention includes forming a recess in a first layer of an ophthalmic lens formed of at least two layers and placing a desired component in the recess to make an ophthalmic lens with the component embedded between the two layers.

The method of the present invention may be based on the following two general approaches: - a first approach using a combination of a special male mold with a protrusion formed thereon and a normal female mold to form a first layer of an ophthalmic lens with a recess formed therein; - a second approach using a combination of a normal male mold and a special female mold with a protrusion formed thereon to form a first layer of an ophthalmic lens with a recess formed therein.

The only difference between "special mold" and "normal mold" is that a special mold has a protrusion on its surface while a normal mold does not. Otherwise, the molds are not distinguished from each other unless otherwise specified, and their materials and sizes may be identical or different.

While the ophthalmic lens produced in this manner has a two-layered structure, ophthalmic lenses with two or more layers each having an embedded component can be obtained by using molds having varying arrangements of protrusions. While the material or composition for the first and the second layers are described herein as a first, second, third, or fourth ophthalmic lens material, they are not particularly limited and may be identical to or different from each other.

### [1. First forming method of the present invention]

A first method of forming an ophthalmic lens with an embedded component in accordance with the present invention includes at least the following Steps (A1) to (D1) below:
(A1) mating a first male mold with a first female mold with a first ophthalmic lens material injected therein, and subjecting the first ophthalmic lens material to a first copolymerization reaction to form a first layer of the ophthalmic lens on a concave forming surface of the first female mold, wherein the first male mold has a convex forming surface having a protrusion thereon conforming to the shape of a component to be embedded within the ophthalmic lens, and the first female mold has the concave forming surface corresponding to the first male mold;
(B1) separating the first male mold from the first female mold, placing the component to be embedded in a recess in the first layer of the ophthalmic lens that corresponds to the protrusion, and injecting a second ophthalmic lens material onto the concave forming surface of the first female mold;
(C1) mating a second male mold with the first female mold with the second ophthalmic lens material injected therein, the second male mold having a convex forming surface corresponding to the first female mold, and subjecting the second ophthalmic lens material to a second copolymerization reaction to form a second layer of the ophthalmic lens over the concave forming surface of the first female mold; and
(D1) separating the second male mold from the first female mold to obtain the ophthalmic lens having the component embedded between the second layer and the first layer.

In brief, the first method of the present invention provides an ophthalmic lens in the following manner: In Step (A1), a first male mold on which a protrusion conforming to a component to be embedded has been pre-formed is mated with a first female mold in which a first ophthalmic lens material has been injected. The first ophthalmic lens material is then subjected to a first copolymerization reaction to form a first layer. Next, in Step (B1), the first male mold is separated from the first female mold and a component to be embedded is placed in a recess formed in the first layer within the first female mold, the recess corresponding to the protrusion. A second ophthalmic lens material is then injected into the first female mold. Next, in Step (C1), a second male mold is mated with the first female mold and the second ophthalmic lens material is subjected to a second copolymerization reaction to form a second layer over a concave forming surface of the first female mold. Next, in Step (D1), the second male mold is separated from the first female mold to obtain an ophthalmic lens. As described above, in the first method of the present invention, the first male mold corresponds to the special mold having a protrusion thereon whereas the second male mold and the first female mold each correspond to the normal mold without any protrusion. The first and the second ophthalmic lens material may be identical to or different from each other.

According to the method of the present invention, an ophthalmic lens can be achieved that has a component reliably embedded at a desired position within the lens. In the method of the present invention, Steps (A1) to (D1) and Steps (A2) to (D2) do not necessarily have to be performed sequentially; rather, various other steps or processes may be performed between or during steps to the extent that the objectives of the present invention are achieved.

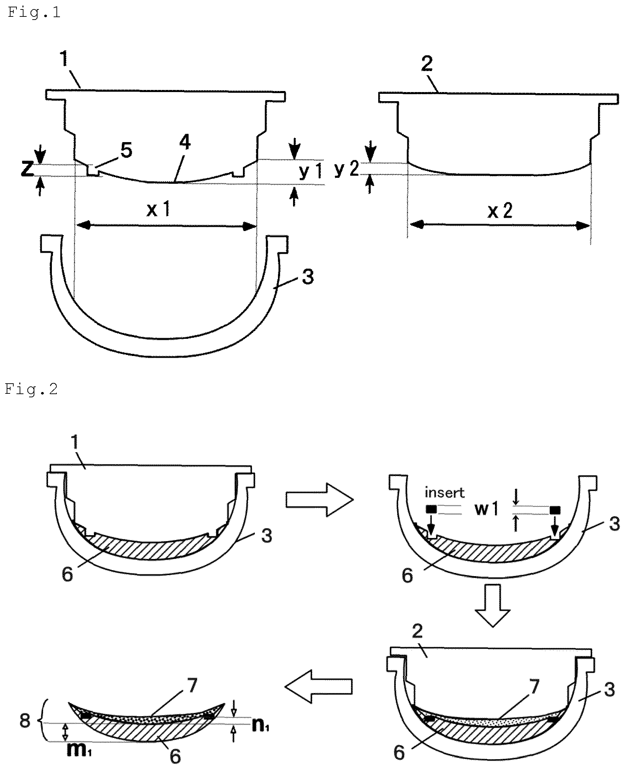

The present invention will now be described specifically with reference to the accompanying drawings. In the present invention, an ophthalmic device is formed in at least two polymerization steps to have a two-layered structure. With reference to FIGS. 1 and 2, in Step (A1) of a first method of the present invention, a first male mold 1 is mated with a first female mold 3 with a first ophthalmic lens material injected therein. The first ophthalmic lens material is then subjected to a copolymerization reaction to form a first layer 6 of an ophthalmic lens on a concave forming surface of the first female mold 3.

The first male mold 1 has a convex forming surface and a protrusion 5 pre-formed on the surface of the mold and conforming to the shape of a component for being embedded within the ophthalmic lens. As used herein, "the surface of the first male mold 1" refers to a surface of the first male mold 1 on the side that mates with the paired first female mold 3. In addition to the protrusion 5, the surface of the first male mold 1 also has an optical area 4. The optical area 4 corresponds to the optical zone of the formed ophthalmic lens. The position of the protrusion 5 is not particularly limited as long as the optical area 4 is not affected; it is formed at a desired position depending on the use and the shape of the component to be embedded. In the present invention, the height z of the protrusion 5 is preferably from 50% to 150% of the thickness w1 of the component to be embedded. The height z of the protrusion 5 corresponds to the depth of the recess in the first layer 6 formed in the subsequent step. The thickness w1 of the component to be embedded refers to the height of the component in the direction along which the component is embedded. As used herein, "the center of the first layer 6" corresponds to the center of the ophthalmic lens 8 formed in the subsequent step and refers to the center of the mold on which the first layer 6 is formed.

The first female mold 3 having the concave forming surface is formed to correspond to the first male mold 1 with the protrusion 5 formed thereon. The diameter of the first male mold 1 and the diameter of the first female mold 3 are the same size x1 and corresponds to the diameter of the ophthalmic lens 8 formed in the subsequent step. The height y1 of the first male mold 1 indicates the vertical height measured from the outer edge of the first layer formed between the first male mold 1 and the first female mold 3 upon mating of the molds to the lens center on the surface of the first layer facing the first male mold 1.

Referring to FIG. 2, the ophthalmic lens material is injected into the forming surface of the first female mold 3. The first female mold 3 is then mated with the forming surface of the first male mold 1 on which the protrusion 5 is formed. The first copolymerization reaction is then carried out. In this manner, the first layer 6 of the ophthalmic lens with a recess corresponding to the shape of the component to be embedded is formed on the surface of the first female mold 3.

In the method of the present invention, the ophthalmic lens materials for forming the two-layered structure may be identical or different for the first and the second layers. Examples of the materials of the ophthalmic lens include monomer components, such as hydrophilic monomers, hydrophobic monomers, silicone monomers and cross-linkable monomers, preferably (meth)acrylate monomers. The material for the ophthalmic lens is properly selected to provide the physical properties of the desired ophthalmic lens. The amount of the monomer component may be any suitable amount that allows the resulting copolymer obtained by the copolymerization reaction to maintain its formability and is not particularly limited. For example, the monomer components may be used in amounts commonly used in the production of contact lenses.

Examples of the hydrophilic (meth)acryl monomer include 2-hydroxyethyl (meth)acrylate (HEMA), 2-hydroxymethyl (meth)acrylate, hydroxypropyl (meth)acrylate, and glycerol (meth)acrylate.

Examples of the hydrophobic (meth)acryl monomer include straight-chained, branched or cyclic alkyl (meth)acrylates, such as methyl (meth)acrylate(MMA), ethyl (meth)acrylate, propyl (meth)acrylate, i-propyl (meth)acrylate, n-butyl (meth)acrylate, i-butyl (meth)acrylate, t-butyl (meth)acrylate, 2-ethylhexyl (meth)acrylate, cyclohexyl (meth)acrylate, benzyl (meth)acrylate, and lauryl acrylate (LA); and fluorine-based (meth)acrylates, such as trifluoroethyl (meth)acrylate, tetrafluoropropyl (meth)acrylate (V-4F), tetrafluoropentyl (meth)acrylate, and hexafluorobutyl (meth)acrylate.

Examples of the silicone (meth)acryl monomer include methacryloxypropylbis(trimethylsiloxy)methylsilane, α,ω-dimethacryloxypropyl polydimethylsiloxane (FM7725), α-methacryloxy-w-butylpolydimethylsiloxane, methacryloxy propyltris(trimethylsiloxy)silane, and tris(trimethylsiloxy)silylpropylmethacrylate (TRIS).

Examples of the cross-linkable (meth)acryl monomer include ethyleneglycol di(meth)acrylate (EDMA), and trimethylol propanetri(meth)acrylate.

Example of the polymerization initiator used in the polymerization of a mixture of the above-described monomers include common radical polymerization initiators including peroxides such as lauroyl peroxide, cumene hydroperoxide, and benzoyl peroxide, and azobis valeronitrile and azobisisobutyronitrile (AIBN). The amount of the polymerization initiator may be any suitable amount for achieving its desired objective and is not particularly limited. For example, the polymerization initiator is used in an amount of about 10 ppm to about 3500 ppm with respect to the total amount of monomers.

The ophthalmic lens material in the present invention may contain a UV-absorber. Specific examples include 2-hydroxy-4-(meth)acryloyloxybenzophenone, 2-hydroxy-4-(meth)acryloyloxy-5-t-butylbenzophenone, 2-(2'-hydroxy-5'-(meth)acryloyloxyethylphenyl)-2H-benzotriazole, 2-(2'-hydroxy-5'-(meth)acryloyloxyethylphenyl)-5-chloro-2H-benzotriazole, 2-hydroxy-4-methacryloyloxymethyl phenylbenzoate, and methacrylic acid 2-[3-(2H-benzotriazole-2-yl)-4-hydroxyphenyl]ethyl (RUVA). The UV-absorber may be added in any suitable amount to provide desired UV absorption ability.

The polymerization initiator is added to a mixture of any combination of the hydrophilic (meth)acryl monomer, the hydrophobic (meth)acryl monomer, the silicone (meth)acryl monomer, and the cross-linkable (meth)acryl monomer and the mixture is stirred to dissolve the monomer components to obtain a monomer mixture.

The resulting monomer mixture is placed in a mold formed of a material such as metal, glass, or plastic and the mold is sealed. The temperature of the mold is then raised to 25°C to 130°C either stepwise or continuously using for example a temperature-controlled bath and maintained for 5 hours to 120 hours to complete the polymerization. UV light, electron beams or gamma-rays may also be used for polymerization. Alternatively, water or an organic solvent may be added to the monomer mixture to carry out solution polymerization.

With reference to FIG. 2, in Step (B1) of the first method of the present invention, the first male mold 1 and the first female mold 3 are separated from one another after formation of the first layer 6 on the first female mold 3 in Step (A1). As a result, a recess corresponding to the shape of the component to be embedded is formed on the surface of the first layer 6 of the ophthalmic lens. The shape of the recess is determined based on the shape of the protrusion 5. Specifically, the height z of the protrusion provides the depth of the recess.

Subsequently, a component to be embedded (i.e., insert) is placed in the recess formed on the first layer 6 in the first female mold 3. A second ophthalmic lens material is then injected into the first female mold 3. It should be noted that the materials do not necessarily have to be added by injection; they may simply be added.

In the present invention, the component to be embedded within the ophthalmic lens may take various desired forms depending on the functions imparted to the contact lens. In forming a fashion contact lens, for example, the component preferably has an annular shape with an iris pattern arranged on it. In forming a contact lens for monitoring and/or examination of a biological function, the component preferably takes the form of a chip that can serve as a sensor for the biological function or may have an annular shape.

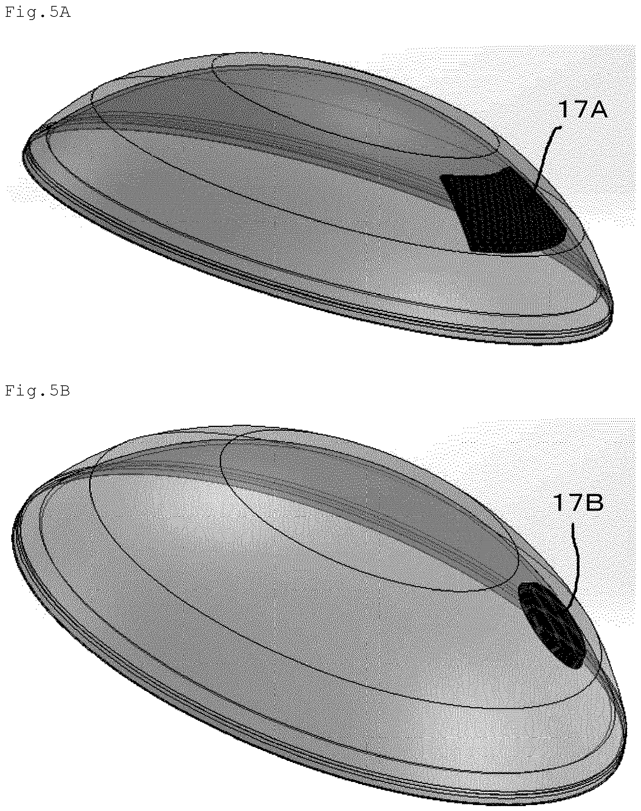

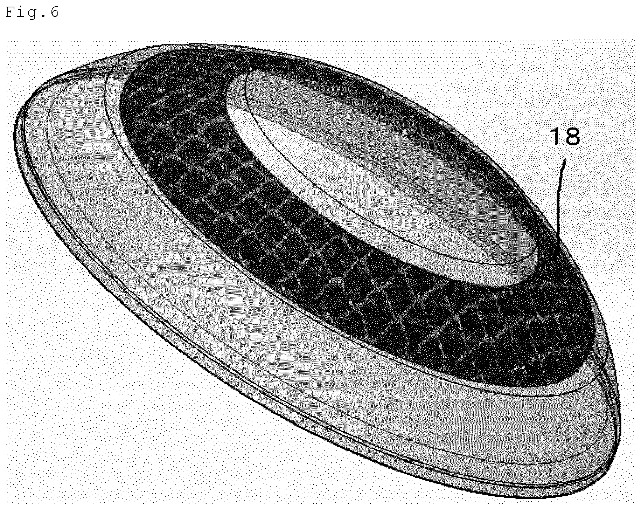

FIG. 5A shows one example of an ophthalmic lens with an embedded measurement device 17A in the form of a chip (rectangular in shape) to serve as a sensor. FIG. 5B shows one example of an ophthalmic lens with an embedded measurement device 17B in the form of a chip (circular in shape) to serve as a sensor. FIG. 6 shows one example of an ophthalmic lens with an embedded annular measurement device 18 to serve as a sensor. A combination of the annular form and the chip form may also be used.

The thickness of the ophthalmic lens at its center is not particularly limited. For example, the thickness at the center may be not greater than 0.5 mm.

The thickness w1 of the component embedded in the ophthalmic lens (also referred to as "components to be embedded") is not particularly limited. The present invention allows, for example, embedding a component with a thickness of 0.001 mm or more, preferably 0.01 mm or more, more preferably 0.1 mm or more, and still more preferably 0.2 mm or more.

According to the invention, the position of the component to be embedded is within the formed lens, and is positioned outside the optical zone. According to the invention, the component to be embedded is positioned such that the center of the component to be embedded is located at the edge part of the surface of the first layer, at 1 mm to 5 mm from the outer edge of the formed ophthalmic lens. According to the method of the present disclosure, when the component to be embedded has a detector portion on one side of the component, the component may be placed to be oriented as desired, for example, in such a manner that the side with the detector portion faces toward the inside of the eye.

The second ophthalmic lens material injected into the first female mold 3 may be identical to or different from the lens material used in the previous Step (A1) and may be properly selected for its type and compositional ratio depending on the ophthalmic lens one wishes to obtain.

With reference to FIGS. 1 and 2, in Step (C1) of the first method of the present invention, a second male mold 2 corresponding to the first female mold 3 is mated with the first female mold 3 with the second ophthalmic lens material injected therein. The second ophthalmic lens material is then subjected to a second copolymerization reaction to form a second layer 7. The second male mold 2 has a convex forming surface without any protrusion like the one formed on the first male mold 1. The diameter x2 of the second male mold 2 is the same as the diameter x1 of the first male mold 1 used in Steps (A1) and (B1). The height y2 of the second male mold 2 indicates the vertical height measured from the contact point between the second male mold 2 and the first female mold 3 that are mated together, or the outer edge of the first layer 6 or the second layer 7 formed therebetween, to the lens center on the surface of the second layer 7 facing the second male mold 2. The height y2 of the second male mold 2 is preferably 50% to 99.9%, more preferably 75% to 99%, and still more preferably 80% to 99% with respect to the height y1 of the first male mold 1 used in Steps (A1) and (B1). This realizes the two-layered structure of the ophthalmic lens.

With reference to FIG. 2, in Step (D1) of the present invention, the second male mold 2 and the first female mold 3 that were mated together in Step (C1) are separated from one another to obtain an ophthalmic lens 8 having the component embedded between the second layer 7 and the first layer 6.

After separation of the second male mold 2 and the first female mold 3, one of the molds that has the attached ophthalmic lens 8 is immersed in a solution to remove the lens from the mold. This gives a desired ophthalmic lens. The separation means is not particularly limited. For example, the separation-by-swelling technique using an organic solvent or a mixture of organic solvent/water may be employed. The organic solvent used in the separation-by-swelling technique is not particularly limited and may be any organic solvent that can undergo solvation and is miscible with water when the lens material contains a silicone-containing monomer. For example, the organic solvent may be a primary or secondary alcohol. Specific examples of the organic solvent used in the removal by swelling include, but are not limited to, methanol, ethanol, propanol, isopropanol, n-butanol, and isobutanol. The organic solvents used in the removal by swelling may be used either alone or as a mixture of two or more solvents. When removed from the ophthalmic lens mold, a silicone-based soft ophthalmic lens from which unreacted components have been removed can be obtained. When the ophthalmic lens material includes a hydrous monomer, physiological saline and phosphate buffer may also be used.

The ophthalmic lens 8 formed to have the above-described construction has a ratio of the thickness m1 at the center of the first layer to the thickness n1 at the center of the second layer in the range of 1:0.1 to 1:25, preferably in the range of 1:0.1 to 1:20. This allows the component to be effectively embedded within the ophthalmic lens. The thickness m1 at the center of the first layer 6 is defined as the distance between the lens center on the convex forming surface of the first male mold 1 and the lens center of the first female mold 3. The thickness n1 at the center of the second layer 7 is defined as the distance between the lens center on the convex forming surface of the second male mold 2 and the lens center of the forming inner surface of the first female mold 3 minus the thickness m1 at the center of the first layer.

The first layer 6 of the ophthalmic lens 8 with the recess for placing the component therein, which is obtained in Steps (A1) and (B1) of the first method of the present invention, selectively adheres to a normal mold with no protrusion, that is, the first female mold 3 in FIG. 2, with the recess left open, when the first male mold 1 is separated from the first female mold 3.

In order for the first layer 6 of the ophthalmic lens to selectively adhere to a desired mold, the affinity of the mold, in this case, the first female mold 3, toward the first layer 6 of the ophthalmic lens is increased. In an embodiment where the first male mold 1 and the first female mold 3 are formed of the same resin, one exemplary process for increasing the affinity involves modifying the surface of the mold to which one wishes to adhere the ophthalmic lens, for example, the surface of the first female mold 3 in FIG. 2, with a physical treatment such as plasma irradiation and corona discharge. Subsequently, the mold combination is used to perform a copolymerization reaction of the ophthalmic lens. The process, however, is not limited to this one. In this manner, the adhesion of the first layer 6 of the ophthalmic lens to the mold 3 is increased, thus allowing the first layer 6 of the ophthalmic lens to selectively adhere to a desired mold. In such instances, the resin may be properly selected from the resins listed below regardless of the nature of the ophthalmic lens.

The resin used to make the molds is not particularly limited; any resin that is inert, and in which the ophthalmic lens material is not soluble can be used, regardless of whether the mold is a special mold with a protrusion or a normal mold with no protrusion, or whether the mold is a male mold or a female mold. Specific examples of the resin include, but are not limited to, polyethylene, polypropylene, ethylene/vinyl acetate, polystyrene, AS resins, ABS resins, polymethylmethacrylate, polyvinyl chloride, cellulose resins, polyacetal, polyamide, polybutylene terephthalate, polyethylene terephthalate, polycarbonate, denatured polyphenylene ether, polyphenylene sulfide, polyether ether ketone, liquid crystal polymers, polysulfone, polyether sulfone, polyarylate, polyamide-imide, polyetherimide, polymethylpentene, phenol resins, urea resins, melamine resins, and epoxy resins. These resins used to make the molds may be used either individually or in combination of two or more resins. The technique for forming the molds may be properly selected from injection molding and other known techniques such as compression molding and vacuum molding. Polypropylene is preferably used for its formability.

In another embodiment where the first male mold 1 and the first female mold 3 are formed of different resins, the mold to which one wishes to adhere the first layer 6 of the ophthalmic lens, that is, normal first female mold 3, is formed of a resin having a high affinity for the first layer 6 of the ophthalmic lens, whereas the corresponding mold, or first male mold 1, is formed of a resin having a low affinity for the ophthalmic lens. Subsequently, the mold combination is used to perform a copolymerization reaction of the ophthalmic lens. This allows the first layer 6 of the ophthalmic lens to selectively adhere to the desired mold, or the first female mold 3.

### [2. Second forming method of the present invention]

As another embodiment of the method of the present invention, a second method of forming an ophthalmic lens with an embedded component in accordance with the present invention includes at least the following Steps (A2) to (D2) below:
(A2) mating a third male mold with a second female mold with a third ophthalmic lens material injected therein, and subjecting the third ophthalmic lens material to a first copolymerization reaction to form a first layer of the ophthalmic lens on a convex forming surface of the third male mold, wherein the second female mold has a concave forming surface having a protrusion thereon conforming to the shape of the component to be embedded within the ophthalmic lens, and the third male mold has the convex forming surface corresponding to the second female mold;
(B2) separating the second female mold from the third male mold, placing the component to be embedded in a recess in the first layer of the ophthalmic lens corresponding to the protrusion, and injecting a fourth ophthalmic lens material onto a concave forming surface of a third female mold that corresponds to the third male mold;
(C2) mating the third male mold with the third female mold with the fourth ophthalmic lens material injected therein, and subjecting the fourth ophthalmic lens material to a second copolymerization reaction to form a second layer of the ophthalmic lens on the first layer of the ophthalmic lens on the convex forming surface of the third male mold; and
(D2) separating the third male mold from the third female mold to obtain the ophthalmic lens with the component embedded between the second layer and the first layer.

Unlike the first method, the second method of the present invention uses a combination of a normal male mold and a special female mold with a protrusion formed thereon to form a first layer with a recess formed therein. Thus, the method is performed in the same manner as the first method of the present invention, except that the mold having a pre-formed protrusion conforming to the shape of the component to be embedded is changed from the first male mold used in the first method of the present invention, to a second female mold with a concave forming surface; that the mold on which to form the first layer is changed from the first female mold to a third male mold with a convex forming surface; that a second material (i.e., fourth ophthalmic lens material) is injected into a third female mold beforehand, and that the third male mold with the first layer is mated with the third female mold having the injected material to perform a second copolymerization reaction. The normal third male mold may be identical to or different from the normal second male mold used in the first method. Also, the normal third female mold may be identical to or different from the normal first female mold used in the first method. The differences from the first method are described in detail below.

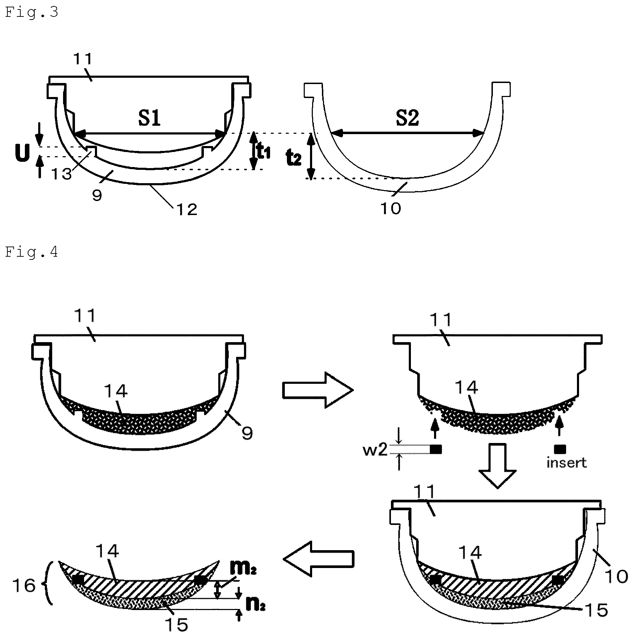

With reference to FIGS. 3 and 4, in Step (A2) of a second method of the present invention, a third male mold 11 is mated with a second female mold 9 with a third ophthalmic lens material injected therein. The third ophthalmic lens material is then subjected to a copolymerization reaction to form a first layer 14 of an ophthalmic lens on a convex forming surface of the third male mold 11.

The second female mold 9 has a concave forming surface and a protrusion 13 formed on the surface of the mold and conforming to the shape of a component for being embedded within the ophthalmic lens. As used herein, "the surface of the second female mold 9" refers to a surface of the second female mold 9 on the side that mates with the paired third male mold 11. In addition to the protrusion 13, the surface of the second female mold 9 also has an optical area 12. The optical area 12 is a similar optical area to the optical area 4. The position of the protrusion 13 is not particularly limited as long as the optical area 12 is not affected; it is formed at a desired position depending on the use and the shape of the component to be embedded. In the present invention, the height u of the protrusion 13 is preferably from 50% to 150% of the thickness w2 of the component to be embedded. The height u of the protrusion 13 corresponds to the depth of the recess in the first layer 14 formed in the subsequent step. The thickness w2 of the component to be embedded refers to the height of the component in the direction along which the component is embedded. As used herein, "the center of the first layer 14" corresponds to the center of the ophthalmic lens 16 formed in the subsequent step and refers to the center of the mold on which the first layer 14 is formed.

The third male mold 11 having the convex forming surface is formed to correspond to the second female mold 9 with the protrusion 13 formed thereon. The diameter of the second female mold 9 and the diameter of the third male mold 11 are the same size s1 and corresponds to the diameter of the ophthalmic lens 16 formed in the subsequent step. The height t1 of the second female mold indicates the vertical height measured from the outer edge of the first layer formed between the second female mold 9 and the third male mold 11 upon mating of the molds to the lens center on the surface of the first layer facing the second female mold 9.

Referring to FIG. 4, the third ophthalmic lens material is injected into the forming surface of the second female mold 9. The second female mold 9 is then mated with the forming surface of the third male mold 11. The first copolymerization reaction is then carried out. In this manner, the first layer 14 of the ophthalmic lens with a recess corresponding to the shape of the component to be embedded is formed on the surface of the third male mold 11. The formation of the first layer by copolymerization reaction is similar to that in the first method described above.

With reference to FIG. 4, in Step (B2) of the second method of the present invention, the second female mold 9 and the third male mold 11 are separated from one another after formation of the first layer 14 on the third male mold 11 in Step (A2). As a result, a recess corresponding to the shape of the component to be embedded is formed on the surface of the first layer 14 of the ophthalmic lens. The shape of the recess is determined based on the shape of the protrusion 13. Specifically, the height u of the protrusion 13 provides the depth of the recess.

Subsequently, a component to be embedded (i.e., an insert) is placed in the recess formed on the first layer 14 on the third male mold 11. A fourth ophthalmic lens material is then injected into a third female mold 10.

The component to be embedded and the ophthalmic lens materials are similar to those used in the first method. Thus, the height w2 of the component to be embedded is similar to w1 described above.

With reference to FIGS. 3 and 4, the third female mold 10 has a concave forming surface that corresponds to the third male mold 11 and does not have any protrusion like the one formed on the first female mold 9. The diameter s2 of the third female mold 10 is the same as the diameter s1 of the second female mold 9 used in Steps (A2) and (B2). The height t2 of the third female mold 10 indicates the vertical height measured from the contact point between the third female mold 10 and the third male mold 11 when mated together, or the outer edge of the first layer 14 or the second layer 15 formed therebetween, to the lens center on the surface of the second layer 15 facing the third female mold 10. The height t2 of the third female mold 10 is preferably 100.1% to 200%, more preferably 101% to 150%, and still more preferably 101% to 125% with respect to the height t1 of the second female mold 9 used in Steps (A2) and (B2). This realizes the two-layered structure of the ophthalmic lens.

With reference to FIG. 4, in Step (D2) of the present invention, the third male mold 11 and the third female mold 10 that were mated together in Step (C2) are separated from one another to obtain an ophthalmic lens 16 having the component embedded between the second layer 15 and the first layer 14.

After separation of the third male mold 11 and the third female mold 10, one of the molds that has the attached ophthalmic lens 16 is immersed in a solution to remove the lens from the mold. This gives a desired ophthalmic lens.

The ophthalmic lens 16 formed to have the above-described construction has a ratio of the thickness m2 at the center of the first layer to the thickness n2 at the center of the second layer in the range of 1:0.1 to 1:25, preferably in the range of 1:0.1 to 1:20. This allows the component to be effectively embedded within the ophthalmic lens. The thickness m2 at the center of the first layer 14 is defined as the distance between the lens center on the convex forming surface of the third male mold 11 and the lens center on the concave forming inner surface of the second female mold 9. The thickness n2 at the center of the second layer 15 is defined as the distance between the lens center on the concave forming surface of the third female mold 10 and the lens center of the convex forming surface of the third male mold 11 minus the thickness m2 at the center of the first layer.

Also in the second method of the present invention, the first layer 14 of the ophthalmic lens 16 with the recess for placing the component therein should selectively adhere to a normal mold with no protrusion, that is, the third male mold 11 in FIG. 4, with the recess left open, when the third male mold 11 is separated from the second female mold 9. In order for the first layer 14 of the ophthalmic lens to selectively adhere to a desired mold, the affinity of the normal mold, in this case, the third male mold 11, toward the first layer 14 of the ophthalmic lens may be increased.

It should be noted that in the first method of the present invention, a layer including the outer side (i.e., the side opposite the eye contacting side) of the resulting ophthalmic lens is first formed as the first layer, followed by the formation of the second layer including the inner side of the ophthalmic lens. Conversely, in the second method of the present invention, the inner side of the ophthalmic lens is first formed as the first layer, followed by the formation of the second layer including the outer side of the ophthalmic lens. This order is not particularly limited and either method may be used to embed the component within the ophthalmic lens in a reliable fashion. Thus, the materials and compositional ratios for the ophthalmic lens may be selected depending on the desired component to be embedded to form an ophthalmic lens having a two-layered structure.

According to the invention, an ophthalmic lens refers therein to a contact lens.

The ophthalmic lens with an embedded component allows precise positioning of the component to be embedded by using a mold that has a pre-formed impression of the component. As described in the Examples, the ophthalmic lens with an embedded component provides improved selective adhesion of the embedded component as well as improved stability without causing shifting or protruding of the embedded component. The ophthalmic lens with an embedded component allows predetermined orientation of complex and delicate components, such as medical devices, and is therefore highly convenient for the wearer.

In addition to the above-described steps, the method of the present invention may include combinations of various other steps known to those skilled in the art as long as the production method of the ophthalmic lens with embedded components can be achieved. While such other steps are not particularly limited, the method of the present invention may, for example, include the following specific steps:
(A1) mating a first male mold with a first female mold with an ophthalmic lens material injected therein, the ophthalmic lens material containing a monomer component being selected from the group consisting of hydrophilic monomers, hydrophobic monomers, silicone monomers, and cross-linkable monomers, and subjecting the ophthalmic lens material to a copolymerization reaction to form a first layer of the ophthalmic lens on a concave forming surface of the first female mold, wherein the first male mold has a convex forming surface having a protrusion thereon that conforms to the shape of an annular component having an iris pattern or an sensor component for a biological function to be embedded within the ophthalmic lens, wherein the first female mold has the concave forming surface corresponding to the first male mold, and wherein the height of the protrusion is 50% to 150% of the thickness of the component to be embedded;
(B1) separating the first male mold from the first female mold, placing the component to be embedded in a recess in the first layer of the ophthalmic lens that corresponds to the protrusion, and injecting a second ophthalmic lens material onto the concave forming surface of the first female mold, the second ophthalmic lens material containing the same or different components as that of the first ophthalmic lens;
(C1) mating a second male mold with the first female mold with the second ophthalmic lens material injected therein, the second male mold having a convex forming surface corresponding to the first female mold, and subjecting the second ophthalmic lens material to a second copolymerization reaction to form a second layer of the ophthalmic lens over the concave forming surface of the first female mold, wherein the height of the second male mold is 50% to 99.9% of the height of the first male mold, and wherein the ratio of the thickness at the center of the first layer to the thickness at the center of the second layer is 1:0.1 to 1:25; and
(D1) separating the second male mold from the first female mold to obtain the ophthalmic lens having the component embedded between the second layer and the first layer, wherein the thickness of the component to be embedded is less than 0.5 mm, and wherein the orientation of the component to be embedded is such that the center of the component to be embedded is positioned 1 mm to 5 mm from the outer edge of the ophthalmic lens.

For example, the method of the present invention can include the following other specific steps:
(A2) mating a third male mold with a second female mold with a third ophthalmic lens material injected therein, the third ophthalmic lens material containing a monomer component being selected from the group consisting of hydrophilic monomers, hydrophobic monomers, silicone monomers, and cross-linkable monomers, and subjecting the third ophthalmic lens material to a first copolymerization reaction to form a first layer of the ophthalmic lens on a convex forming surface of the third male mold, wherein the second female mold has a concave forming surface having a protrusion thereon that conforms to the shape of an annular component having an iris pattern or a sensor component for a biological function to be embedded within the ophthalmic lens, wherein the third male mold has the convex forming surface corresponding to the second female mold, and wherein the height of the protrusion is 50% to 150% of the thickness of the component to be embedded;
(B2) separating the second female mold from the third male mold, placing the component to be embedded in a recess in the first layer of the ophthalmic lens corresponding to the protrusion, and injecting a fourth ophthalmic lens material onto a concave forming surface of a third female mold that corresponds to the third male mold, the fourth ophthalmic lens material containing the same or different components as the third ophthalmic lens material;
(C2) mating the third male mold with the third female mold with the fourth ophthalmic lens material injected therein, and subjecting the fourth ophthalmic lens material to a second copolymerization reaction to form a second layer of the ophthalmic lens over the first layer of the ophthalmic lens on the convex forming surface of the third male mold, wherein the height of the third female mold is 100.1% to 200% of the height of the second female mold, and wherein the ratio of the thickness at the center of the first layer to the thickness at the center of the second layer is 1:0.1 to 1:25; and
(D2) separating the third male mold from the third female mold to obtain the ophthalmic lens having the component embedded between the second layer and the first layer, wherein the thickness of the component to be embedded is less than 0.5 mm, and wherein the orientation of the component to be embedded is such that the center of the component to be embedded is positioned 1 mm to 5 mm from the outer edge of the ophthalmic lens.

The present invention will now be described in further detail with reference to the following Examples, which are not intended to limit the present invention. The present invention may take various forms to the extent that the objectives of the present invention are achieved.

### Examples

### [1. Formation of molds]

Molds each having a shape shown in Tables 1 to 12 were formed by injection molding to provide mold combinations 1 to 12 for use in the present invention.

### (1) Combination 1

Combination 1 corresponds to the combination in the first configuration of the present invention (FIGS. 1 and 2) (Table 1). A combination of a first male mold 1 having a protrusion, a first female mold 3 corresponding to the first male mold 1, and a second male mold 2 corresponding to the female mold and having a height y2 lower than the height y1 of the first male mold 1 was prepared. The diameter x1, x2 were equal for each of the first male mold 1, the first female mold 3, and the second male mold 2. An annular iris pattern was used as the component to be embedded. The protrusion 5 of the first male mold 1 was an annular protrusion conforming to the shape of the component to be embedded and the height z of the protrusion was 133% relative to the thickness w1 of the component to be embedded. The first female mold 3 was subjected to corona discharge treatment.

### (2) Combination 2

Combination 2 corresponds to the combination in the second configuration of the present invention (FIGS. 3 and 4) (Table 2). A combination of a second female mold 9 having a protrusion, a third male mold 11 corresponding to the second female mold 9, and a third female mold 10 corresponding to the male mold and having a height t2 higher than the height t1 of the second female mold 9 was prepared. The diameter s1, s2 was equal for each of the second female mold, the third male mold, and the third female mold. A chip-shaped measurement device 17A shown in FIG. 5A was used as the component to be embedded. The protrusion 13 of the second female mold 9 was a square protrusion and the height u of the protrusion 13 was 150% relative to the thickness w2 of the component to be embedded. The third male mold 11 was subjected to corona discharge treatment.

### (3) Combination 3

Combination 3 corresponds to the combination in the first configuration of the present invention. An annular measurement device 18 shown in FIG. 6 was used as the component to be embedded. The height z of the protrusion was 75% relative to the thickness w1 of the component to be embedded. Otherwise, the combination had the same configuration (Table 3).

### (4) Combination 4

Combination 4 corresponds to the combination in the second configuration of the present invention. The height u of the protrusion was 100% relative to the thickness w2 of the component to be embedded. Otherwise, the combination had the same configuration (Table 4).

### (5) Combination 5

Combination 5 corresponds to the combination in the first configuration of the present invention. The diameter x1 of the first male mold 1 and that of the first female mold 3 were equal to each other whereas the diameter x2 of the second male mold 2 was larger than the diameter of the first male mold 1 and first female mold 3. Otherwise, the combination had the same configuration (Table 5).

### (6) Combination 6

Combination 6 corresponds to the combination in the second configuration of the present invention. The diameter s1 of the second female mold 9 and that of the third male mold 11 were equal to each other whereas the diameter s2 of the third female mold 10 was larger than the diameter of the second female mold 9 and third male mold 11. Otherwise, the combination had the same configuration (Table 6).

### (7) Combination 7

Combination 7 corresponds to the combination in the first configuration of the present invention. The diameter x1 of the first male mold 1 and that of the first female mold 3 were equal to each other whereas the diameter x2 of the second male mold 2 was smaller than the diameter of the first male mold 1 and first female mold 3. Otherwise, the combination had the same configuration (Table 7).

### (8) Combination 8

Combination 8 corresponds to the combination in the second configuration of the present invention. The diameter s1 of the second female mold 9 and that of the third male mold 11 were equal to each other whereas the diameter s2 of the third female mold 10 was smaller than the diameter of the second female mold 9 and third male mold 11. Otherwise, the combination had the same configuration (Table 8).

### (9) Combination 9

Combination 9 corresponds to the combination in the first configuration of the present invention with the height y2 of the second male mold 2 being higher than the height y1 of the first male mold 1. Otherwise, the combination had the same configuration (Table 9).

### (10) Combination 10

Combination 10 corresponds to the combination in the second configuration of the present invention with the height t2 of the third female mold 10 being lower than the height t1 of the second female mold 9. The height z of the protrusion was 100% relative to the thickness w2 of the component to be embedded. Otherwise, the combination had the same configuration (Table 10).

### (11) Combination 11

Combination 11 corresponds to the combination in the first configuration of the present invention. The combination had the same configuration, except that corona discharge treatment was not performed (Table 11).

### (12) Combination 12

Combination 12 corresponds to the combination in the first configuration of the present invention. The combination had the same configuration, except that no protrusion was formed on the first male mold 1 and a chip-shaped measurement device 17A shown in FIG. 5A was used as the component to be embedded (Table 12).

### [2. Preparation of ophthalmic lens materials]

The components were weighed in the respective amounts (wt%) shown in Table 13, stirred for 30 min, and mixed until uniform to prepare a formulation. The amounts of AIBN and RUVA indicate external amounts added with respect to the total amount of the monomers.

### [3. Formation of ophthalmic lenses]

### (Example 1) Formation of a same material silicone soft ophthalmic lens

According to Table 14, an ophthalmic lens material for a first copolymerization (ophthalmic lens material 1 in Table 13) was dispensed into the corona discharge-treated female mold used in Combination 1 having a concave forming surface (Table 1). The male mold 1 with a convex forming surface was then mated with the female mold (FIGS. 1, 2). The mated molds were warmed stepwise from room temperature to 90°C to complete the first copolymerization reaction, thereby obtaining a first layer of an ophthalmic lens.

The first male mold 1 of Combination 1 was removed and then, using tweezers, a component to be embedded (annular iris pattern) was placed in a recess on the first layer 6 of the ophthalmic lens that had been formed on the first female mold 3 as a result of mating with the first male mold 1 (FIG. 2).

Subsequently, an ophthalmic lens material for second copolymerization shown in Table 14 (ophthalmic lens material 1 in Table 13) was dispensed into the first female mold 3 having the first layer of the ophthalmic material formed thereon. The second male mold 2 was then mated with the female mold. The mated molds were warmed stepwise from room temperature to 90°C to complete the second copolymerization reaction, thereby obtaining a second layer 7 of the ophthalmic lens.

The second male mold 2 was then removed and the first female mold 3 with the ophthalmic lens formed thereon was immersed in a solution shown in Table 14 (2-propanol: i-PrOH) and was left for 30 min at room temperature to remove the ophthalmic lens from the first female mold 3.

The ophthalmic lens removed from the mold was then placed in a vacuum dryer and treated at 80°C under reduced pressure for 150 min to remove the immersion solution, thereby obtaining a desired ophthalmic lens 8.

### (Example 2) Formation of a same material silicone soft ophthalmic lens

A desired ophthalmic lens shown in Table 14 was obtained in the same manner as in the production method of Example 1, except that the ophthalmic lens materials for the first and second copolymerization were replaced by the ophthalmic lens material 2 shown in Table 13.

### (Example 3) Formation of a different material silicone soft ophthalmic lens

A desired ophthalmic lens shown in Table 14 was obtained in the same manner as in the production method of Example 1, except that the ophthalmic lens materials for the first and second copolymerization were replaced by the ophthalmic lens materials 1 and 2 shown in Table 13, respectively and the solvent was replaced by ethanol (EtOH).

### (Example 4) Formation of a different material silicone soft ophthalmic lens

According to Table 14, an ophthalmic lens material for the first copolymerization (ophthalmic lens material 2 in Table 13) was dispensed into the second female mold 9 used in Combination 2 having a concave forming surface (Table 2). The corona discharge-treated third male mold 11 with a convex forming surface was then mated with the female mold (FIGS. 3, 4). The mated molds were warmed stepwise from room temperature to 90°C to complete the first copolymerization reaction, thereby obtaining a first layer 14 of an ophthalmic lens.

The second female mold 9 of Combination 2 was removed from the third male mold 11 and then, using tweezers, a component to be embedded (chip-shaped measurement device 17A) was placed in a recess on the first layer 14 of the ophthalmic lens that had been formed on the third male mold 11 as a result of mating with the second female mold 9 (FIG. 4).

Subsequently, an ophthalmic lens material for second copolymerization shown in Table 14 (ophthalmic lens material 1 in Table 13) was dispensed into the third female mold 10 used in Combination 2 having a concave forming surface. The third male mold 11 with the first layer 14 of the ophthalmic lens formed thereon was then mated with the female mold. The mated molds were warmed stepwise from room temperature to 90°C to complete the second copolymerization reaction, thereby obtaining a second layer 15 of the ophthalmic lens.

The third female mold 10 was then removed and the third male mold 11 with the ophthalmic lens formed thereon was immersed in a solution shown in Table 14 (i-PrOH/ EtOH) and was left for 30 min at room temperature to remove the ophthalmic lens from the third male mold 11.

The ophthalmic lens removed from the mold was then placed in a vacuum dryer and treated at 80°C under reduced pressure for 150 min to remove the immersion solution, thereby obtaining a desired ophthalmic lens 16 (FIG. 5A).

### (Example 5) Formation of a same material silicone soft ophthalmic lens

A desired ophthalmic lens shown in Table 14 was obtained in the same manner as in the production method of Example 1, except that the molds used were replaced by Combination 3 in Table 3 (FIG. 6).

### (Example 6) Formation of a same material silicone soft ophthalmic lens

A desired ophthalmic lens shown in Table 14 was obtained in the same manner as in the production method of Example 2, except that the molds used were replaced by Combination 4 in Table 4.

### (Example 7) Formation of a same material hydrous soft ophthalmic lens

A desired ophthalmic lens shown in Table 14 was obtained in the same manner as in the production method of Example 1, except that the ophthalmic lens materials for the first and second copolymerization were replaced by the ophthalmic lens material 3 shown in Table 13 and the solvent was replaced by physiological saline.

### (Example 8) Formation of a same material hydrous soft ophthalmic lens

A desired ophthalmic lens shown in Table 14 was obtained in the same manner as in the production method of Example 4, except that the ophthalmic lens materials for the first and second copolymerization were replaced by the ophthalmic lens material 4 shown in Table 13 and the solvent was replaced by phosphate buffer.

### (Comparative Example 1) Formation of a same material silicone soft ophthalmic lens molded by molds with different diameters

A desired ophthalmic lens shown in Table 15 was obtained in the same manner as in Example 1, except that the molds used were replaced by Combination 5 in Table 5.

### (Comparative Example 2) Formation of a same material silicone soft ophthalmic lens molded by molds with different diameters

A desired ophthalmic lens shown in Table 15 was obtained in the same manner as in Example 4, except that the molds used were replaced by Combination 6 in Table 6, the ophthalmic lens material for the second copolymerization was replaced by the ophthalmic lens material 2 shown in Table 13, and the solvent was replaced by i-PrOH.

### (Comparative Example 3) Formation of a different material silicone soft ophthalmic lens molded by molds with different diameters

A desired ophthalmic lens shown in Table 15 was obtained in the same manner as in Example 3, except that the molds used were replaced by Combination 7 in Table 7.

### (Comparative Example 4) Formation of a different material silicone soft ophthalmic lens molded by molds with different diameters

A desired ophthalmic lens shown in Table 15 was obtained in the same manner as in Example 4, except that the molds used were replaced by Combination 8 in Table 8 and the solvent was replaced by i-PrOH.

### (Comparative Example 5) Formation of a same material silicone soft ophthalmic lens molded by molds with different heights

A desired ophthalmic lens shown in Table 15 was obtained in the same manner as in Example 1, except that the molds used were replaced by Combination 9 in Table 9 and the solvent was replaced by EtOH.

### (Comparative Example 6) Formation of a same material silicone soft ophthalmic lens molded by molds with different heights

A desired ophthalmic lens shown in Table 15 was obtained in the same manner as in Example 2, except that the molds used were replaced by Combination 10 in Table 10 and the solvent was replaced by i-PrOH/ EtOH.

### (Reference Example 1) Formation of a different material silicone soft ophthalmic lens molded by molds without corona discharge treatment

A desired ophthalmic lens shown in Table 15 was obtained in the same manner as in Example 3, except that the molds used were replaced by Combination 11 in Table 11 and no corona discharge treatment was performed.

### (Reference Example 2) Formation of a same material silicone soft ophthalmic lens molded by molds with no protrusion formed thereon

A desired ophthalmic lens shown in Table 15 was obtained in the same manner as in Example 2, except that the molds used were replaced by Combination 12 in Table 12 and no protrusion was formed on the molds.

### [4. Evaluation of ophthalmic lenses]

Twenty samples each were subjected to the ophthalmic lens-forming process according to each of the methods of Examples 1 to 8, Comparative Examples 1 to 6, and Reference Examples 1 and 2. The samples were evaluated according to the methods below.

### (1) Selective adhesion to desired one of the molds

Upon separation of the male and female molds following completion of the first copolymerization reaction, the proportion of the first layers of the ophthalmic lenses that were adhered to the mold with no protrusion was determined visually. "++" indicates that the number of the first layers adhered to the desired mold was in the range of 16 to 20. "+" indicates that the number of the first layers adhered to the desired mold was in the range of 10 to 15. "x" indicates that the number of the first layers adhered to the desired mold was in the range of 0 to 9.

### (2) Shifting of the embedded component from the desired position

Each sample of the ophthalmic lens in which the first layer had adhered to the mold with no protrusion was subjected to a second copolymerization reaction. The resulting ophthalmic lenses were visually observed and the number of the lenses that showed component shifting was rated as the proportion relative to the number of the lenses subjected to the second copolymerization reaction. "++" indicates that less than 20% showed shifting. "+" indicates that 20% to less than 60% showed shifting. "x" indicates that 60% or more showed shifting.

### (3) Exposure of the embedded component from within the ophthalmic lens

Each of samples of the ophthalmic lens in which the first layer was adhered to the mold with no protrusion was subjected to a second copolymerization reaction. The resulting ophthalmic lenses were visually observed and the number of the lenses that showed exposure of a portion of the component on the surface of the ophthalmic lens was rated as the proportion relative to the number of the lenses subjected to the second copolymerization reaction. "++" indicates that less than 20% showed exposure. "+" indicates that 20% to less than 60% showed exposure. "x" indicates that 60% or more showed exposure.

For Examples 1 to 8, the selective adhesion to the desired mold was good and the resulting ophthalmic lenses showed a low occurrence of shifting of the embedded component as well as a low occurrence of exposure, thus indicating good results.

For Comparative Examples 1 to 4, the insufficient mating between the male mold and the female mold upon the second copolymerization reaction resulted in defective shapes of the edge of the resulting ophthalmic lens, which was not ideal for ophthalmic lenses.

For Comparative Examples 5, 6, the selective adhesion to the desired mold was good and the occurrence of shifting of the embedded components was low. However, the insufficient thickness of the second layer led to a high occurrence of exposure of the embedded component on the surface of the ophthalmic lens, making it difficult for the lens to be used as an ophthalmic lens.

Reference Example 1 showed poor selective adhesion to the desired mold and less desired ophthalmic lenses were obtained.

Reference Example 2, which did not have any recess in which to place the component to be embedded, showed displacement of the component caused by the pressure exerted upon mating of the second male mold, which led to a high occurrence of shifting.

**[Table 1]**

| Combination 1 | | |
|---|---|---|
| First male mold | Diameter (Lens area) | 14.5 mm |
| | Height | 8.52 mm |
| | Shape of protrusion | annular |
| | Height | 0.04 mm |
| | (Ratio of thickness of embedded component) | 133% |
| | (Ratio of first layer thickness) | 53.3% |
| | Position from outer edge | 4.5mm |
| | Material | polypropylene |
| First female mold | Diameter (lens area) | 14.5 mm |
| | Height | 8.60 mm |
| | Material | polypropylene |
| | Corona discharge | Yes |
| Second male mold | Diameter (lens area) | 14.5 mm |
| | Height | 8.47 mm |
| | Material | polypropylene |
| Embedded component | Shape | annular iris pattern |
| | Thickness | 0.03 mm |

**[Table 2]**

| Combination 2 | | |
|---|---|---|
| Third male mold | Diameter (lens area) | 14.0mm |
| | Height | 8.60 mm |
| | Material | polypropylene |
| | Corona discharge | Yes |
| Second female mold | Diameter (Lens area) | 14.0 mm |
| | Height | 8.65 mm |
| | Shape of protrusion | square |
| | Height | 0.03 mm |
| | (Ratio of thickness of embedded component) | 150% |
| | (Ratio of first layer thickness) | 60.0% |
| | Position from outer edge | 2.5 mm |
| | Material | polypropylene |
| Third female mold | Diameter (lens area) | 14.0 mm |
| | Height | 8.75 mm |
| | Material | polypropylene |
| Embedded component | Shape | chip-shaped measurement device |
| | Thickness | 0.02 mm |

**[Table 3]**

| Combination 3 | | |
|---|---|---|
| First male mold | Diameter (Lens area) | 14.3 mm |
| | Height | 8.70 mm |
| | Shape of protrusion | annular |
| | Height | 0.15 mm |
| | (Ratio of thickness of embedded component) | 75% |
| | (Ratio of first layer thickness) | 3000 |
| | Position from outer edge | 1.5mm |
| | Material | polypropylene |
| First female mold | Diameter (lens area) | 14.3 mm |
| | Height | 8.75 mm |
| | Material | polypropylene |
| | Corona discharge | Yes |
| Second male mold | Diameter (lens area) | 14.3 mm |
| | Height | 8.63 mm |
| | Material | polypropylene |
| Embedded component | Shape | annular iris pattern |
| | Thickness | 0.2 mm |

**[Table 4]**

| Combination 4 | | |
|---|---|---|
| Third male mold | Diameter (lens area) | 14.2 mm |
| | Height | 8.65 mm |
| | Material | polypropylene |
| | Corona discharge | Yes |
| Second female mold | Diameter (Lens area) | 14.2 mm |
| | Height | 8.70 mm |
| | Shape of protrusion | square |
| | Height | 0.1 mm |
| | (Ratio of thickness of embedded component) | 100% |
| | (Ratio of first layer thickness) | 200% |
| | Position from outer edge | 1.2 mm |
| | Material | polypropylene |
| Third female mold | Diameter (lens area) | 14.2 mm |
| | Height | 8.73 mm |
| | Material | polypropylene |
| Embedded component | Shape | chip-shaped measurement device |
| | Thickness | 0.1 mm |

**[Table 5]**

| Combination 5 | | |
|---|---|---|
| First male mold | Diameter (Lens area) | 14.3 mm |
| | Height | 8.69 mm |
| | Shape of protrusion | annular |
| | Height | 0.045 mm |
| | (Ratio of thickness of embedded component) | 113% |
| | (Ratio of first layer thickness) | 75% |
| | Position from outer edge | 4.0 mm |
| | Material | polypropylene |
| First female mold | Diameter (lens area) | 14.3 mm |
| | Height | 8.75 mm |
| | Material | polypropylene |
| | Corona discharge | Yes |
| Second male mold | Diameter (lens area) | 14.7 mm |
| | Height | 8.64 mm |
| | Material | polypropylene |
| Embedded component | Shape | annular iris pattern |
| | Thickness | 0.04 mm |

**[Table 6]**

| Combination 6 | | |
|---|---|---|
| Third male mold | Diameter (lens area) | 14.2 mm |
| | Height | 8.65 mm |
| | Material | polypropylene |
| | Corona discharge | Yes |
| Second female mold | Diameter (Lens area) | 14.2 mm |
| | Height | 8.70 mm |
| | Shape of protrusion | square |
| | Height | 0.028 mm |
| | (Ratio of thickness of embedded component) | 140% |
| | (Ratio of first layer thickness) | 56% |
| | Position from outer edge | 2.0 mm |
| | Material | polypropylene |
| Third female mold | Diameter (lens area) | 14.4 mm |
| | Height | 8.76 mm |
| | Material | polypropylene |
| Embedded component | Shape | chip-shaped measurement device |
| | Thickness | 0.02 mm |

**[Table 7]**

| Combination 7 | | |
|---|---|---|
| First male mold | Diameter (Lens area) | 14.3 mm |
| | Height | 8.69 mm |
| | Shape of protrusion | annular |
| | Height | 0.045 mm |
| | (Ratio of thickness of embedded component) | 113% |
| | (Ratio of first layer thickness) | 75% |
| | Position from outer edge | 4.0 mm |
| | Material | polypropylene |
| First female mold | Diameter (lens area) | 14.3 mm |
| | Height | 8.75 mm |
| | Material | polypropylene |
| | Corona discharge | Yes |
| Second male mold | Diameter (lens area) | 13.9 mm |
| | Height | 8.64 mm |
| | Material | polypropylene |
| Embedded component | Shape | annular iris pattern |
| | Thickness | 0.04 mm |

**[Table 8]**

| Combination 8 | | |
|---|---|---|
| Third male mold | Diameter (lens area) | 14.2 mm |
| | Height | 8.65 mm |
| | Material | polypropylene |
| | Corona discharge | Yes |
| Second female mold | Diameter (Lens area) | 14.2 mm |
| | Height | 8.70 mm |
| | Shape of protrusion | square |
| | Height | 0.028 mm |
| | (Ratio of thickness of embedded component) | 140% |
| | (Ratio of first layer thickness) | 56% |
| | Position from outer edge | 2.0 mm |
| | Material | polypropylene |
| Third female mold | Diameter (lens area) | 13.9 mm |
| | Height | 8.76 mm |
| | Material | polypropylene |
| Embedded component | Shape | chip-shaped measurement device |
| | Thickness | 0.02 mm |

**[Table 9]**

| Combination 9 | | |
|---|---|---|
| First male mold | Diameter (Lens area) | 14.3 mm |
| | Height | 8.69 mm |
| | Shape of protrusion | annular |
| | Height | 0.06 mm |
| | (Ratio of thickness of embedded component) | 102% |
| | (Ratio of first layer thickness) | 80% |
| | Position from outer edge | 4.0 mm |
| | Material | polypropylene |
| First female mold | Diameter (lens area) | 14.3 mm |
| | Height | 8.77 mm |
| | Material | polypropylene |
| | Corona discharge | Yes |
| Second male mold | Diameter (lens area) | 14.3 mm |
| | Height | 8.71 mm |
| | Material | polypropylene |
| Embedded component | Shape | annular iris pattern |
| | Thickness | 0.059 mm |

**[Table 10]**

| Combination 10 | | |
|---|---|---|
| Third male mold | Diameter (lens area) | 14.2 mm |
| | Height | 8.65 mm |
| | Material | polypropylene |
| | Corona discharge | Yes |
| Second female mold | Diameter (Lens area) | 14.2 mm |
| | Height | 8.70 mm |
| | Shape of protrusion | square |
| | Height | 0.03 mm |
| | (Ratio of thickness of embedded component) | 100% |
| | (Ratio of first layer thickness) | 60% |
| | Position from outer edge | 2.0 mm |
| | Material | polypropylene |
| Third female mold | Diameter (lens area) | 14.2 mm |
| | Height | 8.68 mm |
| | Material | polypropylene |
| Embedded component | Shape | chip-shaped measurement device |
| | Thickness | 0.03 mm |

**[Table 11]**

| Combination 11 | | |
|---|---|---|
| First male mold | Diameter (Lens area) | 14.5 mm |
| | Height | 8.52 mm |
| | Shape of protrusion | annular |
| | Height | 0.04 mm |
| | (Ratio of thickness of embedded component) | 133% |
| | (Ratio of first layer thickness) | 53.3% |
| | Position from outer edge | 4.5mm |
| | Material | polypropylene |
| | Corona discharge | No |
| First female mold | Diameter (lens area) | 14.5 mm |
| | Height | 8.60 mm |
| | Material | polypropylene |
| Second male mold | Diameter (lens area) | 14.5 mm |
| | Height | 8.47 mm |
| | Material | polypropylene |
| Embedded component | Shape | annular iris pattern |
| | Thickness | 0.03 mm |

**[Table 12]**

| Combination 12 | | | |
|---|---|---|---|
| First male mold | Diameter (Lens area) | | 14.5 mm |
| | Height | | 8.52 mm |
| | Shape of protrusion | | No |
| | Material | | polypropylene |
| | Corona discharge | | Yes |
| First female mold | Diameter (lens area) | | 14.5 mm |
| | Height | | 8.60 mm |
| | Material | | polypropylene |
| Second male mold | Diameter (lens area) | | 14.5 mm |
| | Height | | 8.47 mm |
| | Material | | polypropylene |
| Embedded component | Shape device | chip-shaped measurement | |
| | Thickness | | 0.02 mm |

**[Table 13]**

| | Ophthalmic lens material | | | |
|---|---|---|---|---|
| | 1 | 2 | 3 | 4 |
| FM7725 (%) | 46.5 | 50 | | |
| TRIS (%) | 40 | 30 | | 30 |
| LA (%) | 10 | | | |
| V-4F (%) | | 19.5 | | |
| MMA (%) | 3 | | | |
| HEMA (%) | | | 98.5 | 69.5 |
| MAA (%) | | | 1 | |
| EDMA (%) | 0.5 | 0.5 | 0.5 | 0.5 |
| AIBN (ppm) | 3000 | 3000 | 3000 | 3000 |
| RUVA (ppm) | 3000 | 3000 | 3000 | 3000 |

### Abbreviations:

FM7725; α,ω-dimethacryloxypropyl poly(dimethylsiloxane) MW = 15000 (JNC)
TRIS; Tris(trimethylsiloxy)silyl propyl methacrylate
LA; Lauryl acrylate
V-4F; 2,2,3,3-tetrafluoropropyl acrylate
MMA; methyl methacrylate
HEMA; hydroxyethyl methacrylate
MAA; methyl acrylamide
EDMA; ethylene glycol dimethacrylate
AIBN; 2,2'-azobisbutyronitrile
RUVA; 2-[3-(2H-benzotriazole-2-yl)-4-hydroxyphenyl]ethyl methacrylate

**[Table 14]**

| | | **Ex. 1** | **Ex. 2** | **Ex. 3** | **Ex. 4** | **Ex. 5** | **Ex. 6** | **Ex. 7** | **Ex. 8** |
|---|---|---|---|---|---|---|---|---|---|
| **Conditions for forming ophthalmic lens** | **Mold Combination** | **1** | **1** | **1** | **2** | **3** | **4** | **1** | **2** |
| | **1st copolymeriza tion ophthalmic lens material** | **1** | **2** | **1** | **2** | **1** | **2** | **3** | **4** |
| | **2nd copolymeriza tion ophthalmic lens material** | **1** | **2** | **2** | **1** | **1** | **2** | **3** | **4** |
| | **Solvent** | **i-PrOH** | **i-PrOH** | **EtOH** | **i-PrOH/ EtOH** | **i-PrOH** | **i-PrOH** | **Phys. saline** | **Phos. buffer** |
| **Ratings of ophthalmic lens** | **1st layer thickness (mm)** | **0.075** | **0.05** | **0.075** | **0.05** | **0.05** | **0.05** | **0.075** | **0.05** |
| | **2nd layer thickness (mm)** | **0.04** | **0.045** | **0.04** | **0.045** | **0.07** | **0.03** | **0.04** | **0.045** |
| | **Selective adhesion** | **++ (18)** | **++ (17)** | **++ (18)** | **++ (16)** | **++ (17)** | **++ (19)** | **++ (17)** | **++ (18)** |
| | **Shift of embedded component** | **++ (0/0%)** | **++ (0/0%)** | **++ (0/0%)** | **++ (0/0%)** | **++ (0/0%)** | **++ (1/5.3 %)** | **++ (0/0%)** | **++ (1/5.6 %)** |
| | **Exposure of embedded component** | **++ (0/0%)** | **++ (0/0%)** | **++ (0/0%)** | **++ (0/0%)** | **++ (1/5.9 %)** | **++ (0/0%)** | **++ (1/5.9 %)** | **++ (0/0%)** |

**[Table 15]**

| | | **Com. Ex. 1** | **Com. Ex. 2** | **Com. Ex. 3** | **Com. Ex. 4** | **Com. Ex. 5** | **Com. Ex. 6** | **Ref. Ex. 1** | **Ref. Ex. 2** |
|---|---|---|---|---|---|---|---|---|---|
| **Conditions for forming ophthalmic lens** | **Mold Combination** | **5** | **6** | **7** | **8** | **9** | **10** | **11** | **12** |
| | **1st copolymeriza tion ophthalmic lens material** | **1** | **2** | **1** | **2** | **1** | **2** | **1** | **2** |
| | **2nd copolymeriza tion ophthalmic lens material** | **1** | **2** | **2** | **1** | **1** | **2** | **2** | **2** |
| | **Solvent** | **i-PrOH** | **i-PrOH** | **EtOH** | **i-PrOH** | **EtOH** | **i-PrOH/ EtOH** | **i-PrOH/ EtOH** | **Phos. buffer** |
| **Ratings of ophthalmic lens** | **1st layer thickness (mm)** | **defecti ve shape in the edge (by burrs)** | **defecti ve shape in the edge (by burrs)** | **defecti ve shape in the edge (by burrs)** | **defecti ve shape in the edge (by burrs)** | **0.075** | **0.05** | **0.075** | **0.075** |
| | **2nd layer thickness (mm)** | | | | | **-** | **-** | **0.04** | **0.04** |
| | **Selective adhesion** | **++ (16)** | **++ (17)** | **++ (17)** | **++ (18)** | **++ (18)** | **++ (16)** | **x (7)** | **++ (17)** |
| | **Shift of embedded component** | - | **-** | **-** | **-** | **++(0/0 %)** | **++ (1/6. 3%)** | **++(1/14 .2%)** | **x (15/88 %)** |
| | **Exposure of embedded component** | **-** | **-** | **-** | **-** | **x (16/89 %)** | **x (15/94 %)** | **++ (0/0%)** | **++ (3/17.6 %)** |

### Reference Numerals

1: First male mold in the first configuration
2: Second male mold in the first configuration
3: First female mold in the first configuration
4: Optical area in the first configuration
5: Protrusion in the first configuration
6: First layer of the ophthalmic lens in the first configuration
7: Second layer of the ophthalmic lens in the first configuration
8: Ophthalmic lens in the first configuration
x1: Diameter of the first male mold in the first configuration
x2: Diameter of the second male mold in the first configuration
y1: Height of the first male mold in the first configuration
y2: Height of the second male mold in the first configuration
z: Height of the protrusion in the first configuration
m1: Thickness of the first layer of the ophthalmic lens in the first configuration
n1: Thickness of the second layer of the ophthalmic lens in the first configuration
w1: Thickness of the component to be embedded in the first configuration
9: Second female mold in the second configuration
10: Third female mold in the second configuration
11: Third male mold in the second configuration
12: Optical area in the second configuration
13: Protrusion in the second configuration
14: First layer of the ophthalmic lens in the second configuration
15: Second layer of the ophthalmic lens in the second configuration
16: Ophthalmic lens in the second configuration
s1: Diameter of the second female mold in the second configuration
s2: Diameter of the third female mold in the second configuration
t1: Height of the second female mold in the second configuration
t2: Height of the third female mold in in the second configuration
u: Height of the protrusion the second configuration
m2: Thickness of the first layer of the ophthalmic lens in the second configuration
n2: Thickness of the second layer of the ophthalmic lens in the second configuration
w2: Thickness of the component to be embedded in the second configuration
17A: Chip-shaped (square) measurement device
17B: Chip-shaped (circular) measurement device
18: annular measurement device

### Industrial Applicability

The ophthalmic lens obtained by the above-described production methods allows secure positioning of an embedded component, a foreign object, by pre-forming on a mold a protrusion to form a receptacle for the component to be embedded. Accordingly, the present invention enables provision of contact lenses for use in a variety of fashion or medical applications.

## Claims

1. A method of producing an ophthalmic lens having a component embedded in the ophthalmic lens (8), comprising the steps of:
(A1) mating a first male mold (1) with a first female mold (3) with a first ophthalmic lens material injected therein, and subjecting the first ophthalmic lens material to a first copolymerization reaction to form a first layer (6) of the ophthalmic lens on a concave forming surface of said first female mold, wherein said first male mold has a convex forming surface having a protrusion (5) thereon conforming to the shape of the component within the ophthalmic lens, and said first female mold has the concave forming surface corresponding to said first male mold;
(B1) separating said first male mold from said first female mold, placing said component in a recess in said first layer of the ophthalmic lens that corresponds to said protrusion, and injecting a second ophthalmic lens material onto the concave forming surface of said first female mold;
(C1) mating a second male mold (2) with said first female mold with said second ophthalmic lens material injected therein, said second male mold having a convex forming surface corresponding to said first female mold, and subjecting said second ophthalmic lens material to a second copolymerization reaction to form a second layer (7) of the ophthalmic lens over the concave forming surface of said first female mold; and
(D1) separating said second male mold from said first female mold to obtain the ophthalmic lens (8) having said component embedded between said second layer and the first layer,
wherein the ophthalmic lens is a contact lens, **characterized in that** the component is located outside the optical zone and positioned such that the center of the component is located at the edge part of the surface of the first layer at 1mm to 5mm from an outer edge of the formed ophthalmic lens (8) .

2. A method of producing an ophthalmic lens (16) having a component embedded in the ophthalmic lens, comprising the steps of:
(A2) mating a third male mold (11) with a second female mold (9) with a third ophthalmic lens material injected therein, and subjecting the third ophthalmic lens material to a first copolymerization reaction to form a first layer (14) of the ophthalmic lens on a convex forming surface of said third male mold, wherein said second female mold has a concave forming surface having a protrusion (13) thereon conforming to the shape of the component within the ophthalmic lens, and said third male mold has the convex forming surface corresponding to said second female mold;
(B2) separating said second female mold from said third male mold, placing said component in a recess in said first layer of the ophthalmic lens corresponding to said protrusion, and inj ecting a fourth ophthalmic lens material onto a concave forming surface of a third female mold (10) that corresponds to said third male mold;
(C2) mating said third male mold with said third female mold with the fourth ophthalmic lens material injected therein, and subjecting said fourth ophthalmic lens material to a second copolymerization reaction to form a second layer (15) of the ophthalmic lens on said first layer of the ophthalmic lens on the convex forming surface of said third male mold; and
(D2) separating said third male mold from said third female mold to obtain the ophthalmic lens (16) with said component embedded between said second layer and said first layer,
wherein the ophthalmic lens is a contact lens, **characterized in that** the component is located outside the optical zone and positioned such that the center of the component is located at the edge part of the surface of the first layer at 1mm to 5mm from an outer edge of the formed ophthalmic lens (16).

3. The method of producing an ophthalmic lens having a component embedded in the ophthalmic lens according to claim 1 or 2, wherein a ratio of a thickness of said first layer at its center to a thickness of said second layer at its center is in the range of 1:0.1 to 1:25.

4. The method of producing an ophthalmic lens having a component embedded in the ophthalmic lens according to any one of claim 1 to 3, wherein said first layer and said second layer are formed of the same material.

5. The method of producing an ophthalmic lens having a component embedded in the ophthalmic lens according to any one of claim 1 to 4, wherein said first layer and said second layer are formed of different materials.

## Patentansprüche

1. Verfahren zur Herstellung einer ophthalmischen Linse, die eine in die ophthalmische Linse (8) eingebettete Komponente aufweist, umfassend die Schritte:
(A1) Zusammenfügen einer ersten Patrize (1) mit einer ersten Matrize (3), in die ein erstes ophthalmisches Linsenmaterial eingespritzt wurde, und Unterziehen des ersten ophthalmischen Linsenmaterials einer ersten Copolymerisationsreaktion, um eine erste Schicht (6) der ophthalmischen Linse auf einer konkaven Formfläche der ersten Matrize auszubilden, wobei die erste Patrize eine konvexe Formfläche mit einem Vorsprung (5) darauf aufweist, der mit der Form der Komponente innerhalb der ophthalmischen Linse übereinstimmt, und die erste Matrize die der ersten Patrize entsprechende konkave Formfläche aufweist;
(B1) Trennen der ersten Patrize von der ersten Matrize, Anordnen der Komponente in einer Aussparung in der ersten Schicht der ophthalmischen Linse, die dem Vorsprung entspricht, und Einspritzen eines zweiten ophthalmischen Linsenmaterials auf die konkave Formfläche der ersten Matrize;
(C1) Zusammenfügen einer zweiten Patrize (2) mit der ersten Matrize, in die das zweite ophthalmische Linsenmaterial eingespritzt wurde, wobei die zweite Patrize eine der ersten Matrize entsprechende konvexe Formfläche aufweist, und Unterziehen des zweiten ophthalmischen Linsenmaterials einer zweiten Copolymerisationsreaktion, um eine zweite Schicht (7) der ophthalmischen Linse über der konkaven Formfläche der ersten Matrize auszubilden; und
(D1) Trennen der zweiten Patrize von der ersten Matrize, um die ophthalmische Linse (8) zu erhalten, die die Komponente zwischen der zweiten Schicht und der ersten Schicht eingebettet aufweist, wobei die ophthalmische Linse eine Kontaktlinse ist,
**dadurch gekennzeichnet, dass**
die Komponente sich außerhalb der optischen Zone befindet und so angeordnet ist,
dass sich die Mitte der Komponente am Randteil der Fläche der ersten Schicht in einem Abstand von 1 mm bis 5 mm von einem äußeren Rand der ausgebildeten ophthalmischen Linse (8) befindet.

2. Verfahren zur Herstellung einer ophthalmischen Linse (16), die eine in die ophthalmische Linse eingebettete Komponente aufweist, umfassend die Schritte:
(A2) Zusammenfügen einer dritten Patrize (11) mit einer zweiten Matrize (9), in die ein drittes ophthalmisches Linsenmaterial eingespritzt wurde, und Unterziehen des dritten ophthalmischen Linsenmaterials einer ersten Copolymerisationsreaktion, um eine erste Schicht (14) der ophthalmischen Linse auf einer konvexen Formfläche der dritten Patrize auszubilden, wobei die zweite Matrize eine konkave Formfläche mit einem Vorsprung (13) darauf aufweist, der mit der Form der Komponente innerhalb der ophthalmischen Linse übereinstimmt, und die dritte Patrize die der zweiten Matrize entsprechende konvexe Formfläche aufweist;
(B2) Trennen der zweiten Matrize von der dritten Patrize, Anordnen der Komponente in einer Aussparung in der ersten Schicht der ophthalmischen Linse, die dem Vorsprung entspricht, und Einspritzen eines vierten ophthalmischen Linsenmaterials auf eine konkave Formfläche einer dritten Matrize (10), die der dritten Patrize entspricht;
(C2) Zusammenfügen der dritten Patrize mit der dritten Matrize, in die das vierte ophthalmische Linsenmaterial eingespritzt wurde, und Unterziehen des vierten ophthalmischen Linsenmaterials einer zweiten Copolymerisationsreaktion, um eine zweite Schicht (15) der ophthalmischen Linse auf der ersten Schicht der ophthalmischen Linse auf der konvexen Formfläche der dritten Patrize auszubilden; und
(D2) Trennen der dritten Patrize von der dritten Matrize, um die ophthalmische Linse (16) zu erhalten, wobei die Komponente zwischen der zweiten Schicht und der ersten Schicht eingebettet ist,
wobei die ophthalmische Linse eine Kontaktlinse ist,
**dadurch gekennzeichnet, dass**
die Komponente sich außerhalb der optischen Zone befindet und so angeordnet ist,
dass sich die Mitte der Komponente am Randteil der Fläche der ersten Schicht in einem Abstand von 1 mm bis 5 mm von einem äußeren Rand der ausgebildeten ophthalmischen Linse (16) befindet.

3. Verfahren zur Herstellung einer ophthalmischen Linse, die eine in die ophthalmische Linse eingebettete Komponente aufweist, nach Anspruch 1 oder 2, wobei ein Verhältnis der Dicke der ersten Schicht in ihrer Mitte zu der Dicke der zweiten Schicht in ihrer Mitte im Bereich von 1:0,1 bis 1:25 liegt.

4. Verfahren zur Herstellung einer ophthalmischen Linse, die eine in die ophthalmische Linse eingebettete Komponente aufweist, nach einem der Ansprüche 1 bis 3, wobei die erste Schicht und die zweite Schicht aus dem gleichen Material ausgebildet sind.

5. Verfahren zur Herstellung einer ophthalmischen Linse, die eine in die ophthalmische Linse eingebettete Komponente aufweist, nach einem der Ansprüche 1 bis 4, wobei die erste Schicht und die zweite Schicht aus unterschiedlichen Materialien ausgebildet sind.

## Revendications

1. Procédé de production d'une lentille ophtalmique ayant un composant incorporé dans la lentille ophtalmique (8), comprenant les étapes de :
(A1) conjugaison d'un premier moule mâle (1) avec un premier moule femelle (3) avec un premier matériau de lentille ophtalmique injecté en leur sein, et soumission du premier matériau de lentille ophtalmique à une première réaction de copolymérisation pour former une première couche (6) de la lentille ophtalmique sur une surface de formation concave dudit premier moule femelle, dans lequel ledit premier moule mâle a une surface de formation convexe ayant une saillie (5) sur lui se conformant à la forme du composant au sein de la lentille ophtalmique, et ledit premier moule femelle a la surface de formation concave correspondant audit premier moule mâle ;
(B1) séparation dudit premier moule mâle dudit premier moule femelle, placement dudit composant dans un retrait dans ladite première couche de la lentille ophtalmique qui correspond à ladite saillie, et injection d'un deuxième matériau de lentille ophtalmique sur la surface de formation concave dudit premier moule femelle ;
(C1) conjugaison d'un deuxième moule mâle (2) avec ledit premier moule femelle avec ledit deuxième matériau de lentille ophtalmique injecté en leur sein, ledit deuxième moule mâle ayant une surface de formation convexe correspond audit premier moule femelle, et soumission dudit deuxième matériau de lentille ophtalmique à une seconde réaction de copolymérisation pour former une seconde couche (7) de la lentille ophtalmique sur la surface de formation concave dudit premier moule femelle ; et
(D1) séparation dudit deuxième moule mâle dudit premier moule femelle pour obtenir la lentille ophtalmique (8) ayant ledit composant incorporé entre ladite seconde couche et la première couche,
dans lequel la lentille ophtalmique est une lentille de contact,
**caractérisé en ce que**
le composant est situé en dehors de la zone optique et positionné de sorte que le centre du composant est situé sur la partie de bord de la surface de la première couche à 1 mm à 5 mm d'un bord externe de la lentille ophtalmique formée (8).

2. Procédé de production d'une lentille ophtalmique (16) ayant un composant incorporé dans la lentille ophtalmique, comprenant les étapes de :
(A2) conjugaison d'un troisième moule mâle (11) avec un deuxième moule femelle (9) avec un troisième matériau de lentille ophtalmique injecté en leur sein, et soumission du troisième matériau de lentille ophtalmique à une première réaction de copolymérisation pour former une première couche (14) de la lentille ophtalmique sur une surface de formation convexe dudit troisième moule mâle, dans lequel ledit deuxième moule femelle a une surface de formation concave ayant une saillie (13) sur lui se conformant à la forme du composant au sein de la lentille ophtalmique, et ledit troisième moule mâle a la surface de formation convexe correspondant audit deuxième moule femelle ;
(B2) séparation dudit deuxième moule femelle dudit troisième moule mâle, placement dudit composant dans un retrait dans ladite première couche de la lentille ophtalmique qui correspond à ladite saillie, et injection d'un quatrième matériau de lentille ophtalmique sur une surface de formation concave d'un troisième moule femelle (10) qui correspond audit troisième moule mâle ;
(C2) conjugaison dudit troisième moule mâle avec ledit troisième moule femelle avec ledit quatrième matériau de lentille ophtalmique injecté en leur sein, et soumission dudit quatrième matériau de lentille ophtalmique à une seconde réaction de copolymérisation pour former une seconde couche (15) de la lentille ophtalmique sur ladite première couche de la lentille ophtalmique sur la surface de formation convexe dudit troisième moule mâle ; et
(D2) séparation dudit troisième moule mâle dudit troisième moule femelle pour obtenir la lentille ophtalmique (16) avec ledit composant incorporé entre ladite seconde couche et ladite première couche,
dans lequel la lentille ophtalmique est une lentille de contact,
**caractérisé en ce que**
le composant est situé en dehors de la zone optique et positionné de sorte que le centre du composant est situé sur la partie de bord de la surface de la première couche à 1 mm à 5 mm d'un bord externe de la lentille ophtalmique formée (16).

3. Procédé de production d'une lentille ophtalmique ayant un composant incorporé dans la lentille ophtalmique selon la revendication 1 ou 2, dans lequel un rapport d'une épaisseur de ladite première couche à son centre sur une épaisseur de ladite seconde couche à son centre est dans la plage de 1/0,1 à 1/25.

4. Procédé de production d'une lentille ophtalmique ayant un composant incorporé dans la lentille ophtalmique selon l'une quelconque des revendications 1 à 3, dans lequel ladite première couche et ladite seconde couche sont formées du même matériau.

5. Procédé de production d'une lentille ophtalmique ayant un composant incorporé dans la lentille ophtalmique selon l'une quelconque des revendications 1 à 4, dans lequel ladite première couche et ladite seconde couche sont formées de matériaux différents.
